# EUROPÄISCHE PATENTSCHRIFT

(11) **EP 0 728 735 B1**
(45) Veröffentlichungstag und Bekanntmachung des Hinweises auf die Patenterteilung: **01.04.1998**
(21) Anmeldenummer: 96101950.2
(22) Anmeldetag: 10.02.1996
(51) Int. Cl.: C07C 213/00, C07C 215/08, C07B 55/00

(54) **Verfahren zur Herstellung von racemischen Aminoalkoholen**
Process for the preparation of racemic aminoalcohols
Procédé pour la préparation d'aminoalcools racémiques

(30) Priorität: 21.02.1995 DE 19505992
(43) Veröffentlichungstag der Anmeldung: 28.08.1996
(73) Patentinhaber: Degussa Aktiengesellschaft, 60311 Frankfurt (DE)
(72) Erfinder: Drauz, Karlheinz, Prof. Dr., D-63579 Freigericht (DE); Jahn, Wilfried, D-63571 Gelnhausen (DE); Schwarm, Michael, Dr., D-63755 Alzenau (DE)

(56) Entgegenhaltungen:
- EP-A- 0 485 069
- "HOUBEN-WEYL - METHODEN DER ORGANISCHEN CHEMIE, Bd. E16d" 1992 , GEORG THIEME VERLAG, STUTTGART, DE XP002001608 * Seite 878 - Seite 894 *
- JOURNAL OF ORGANIC CHEMISTRY, Bd. 58, Nr. 13, 1993, EASTON US, Seiten 3568-3571, XP002001481 MARC J. MCKENNON ET AL.: "A Convenient Reduction of Amino Acids and their Derivatives"
- TETRAHEDRON LETTERS, Bd. 33, Nr. 38, 1992, OXFORD GB, Seiten 5517-5518, XP002001482 ATSUSHI ABIKO ET AL.: "An Improved, Convenient procedure for Reduction of Amino Acids to Aminoalcohols: Use of NaBH4 - H2SO4"
- JOURNAL OF ORGANIC CHEMISTRY, Bd. 54, Nr. 15, 21.Juli 1989, EASTON US, Seiten 3750-3751, XP002001606 CHRISTOPHER HOFFMAN ET AL.: "Synthesis of alpha-Amino Acids by reduction of alpha-Oximino Esters..."
- JOURNAL OF THE CHEMICAL SOCIETY, PERKIN TRANSACTIONS 1, 1989, LETCHWORTH GB, Seiten 279-281, XP002001607 YOSHIHIKO MATSUDA ET AL.: "Preparation of alpha-(N-Trimethylsilyl)imino Esters ..."
- PATENT ABSTRACTS OF JAPAN vol. 11, no. 133 (C-418) [2580] , 25.April 1987 & JP-A-61 271258 (KANEGAFUCHI CHEM IND CO LTD ), 1.Dezember 1986,

## Beschreibung

Die Erfindung betrifft ein Verfahren zur Herstellung von racemischen Aminoalkoholen der allgemeinen Formel I, 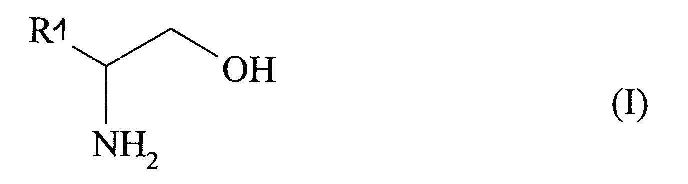 worin R1 ein geradkettiger, verzweigter oder cyclischer Alkyl-, Arylalkyl- oder Arylrest mit bis zu 20 C-Atomen sein kann, der außerdem Heteroatome wie O, N oder S enthalten kann, durch Reduktion von α-Oximinocarbonsäuren oder deren Estern der allgemeinen Formel III, 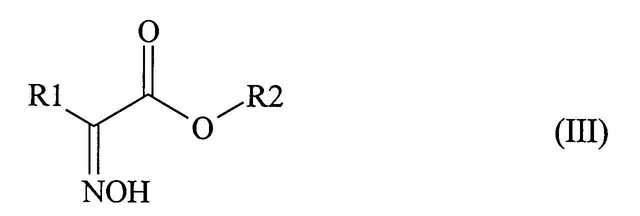 worin R1 die oben angegebene Bedeutung hat und R2 für Wasserstoff oder einen Alkylrest mit bis zu vier C-Atomen steht.

Racemische Aminoalkohole der allgemeinen Formel I sind wichtige Zwischenprodukte bei der Herstellung optisch aktiver Aminoalkohole, die aus den racemischen Verbindungen durch Racematspaltungen zugänglich sind. Diese können entweder enzymatisch (F. Francalani, P. Cesti, W. Cabri, D. Bianchi, T. Martinengo, M. Foà, J. Org. Chem. 1987, 52, 5079; S. Fernández, R. Brieva, F. Rebolledo, V. Gotor, J. Chem. Soc. Perkin Trans. I 1992, 2885;
H. S. Bevinakatti, R. V. Newadkar, Tetrahedron: Asymmetry 1990, 1, 583), durch bevorzugte Kristallisation des einen Enantiomeren (K. Saigo, H. Miura, K. Ishizaki, H. Nohira, Bull. Chem. Soc. Jpn. 1982, 55, 1188) oder klassisch durch Umsetzung mit einer optisch aktiven Säure und fraktionierte Kristallisation der diastereomeren Salzpaare (DE 35 17 108 A1) durchgeführt werden. Die so erhaltenen optisch aktiven Aminoalkohole finden vielfältige Anwendungen z. B. in der Medizin oder Pharmazie als Wirkstoffe oder Zwischenprodukte sowie als chirale Induktoren oder Katalysatoren in der asymmetrischen Synthese.

Die benötigten racemischen Aminoalkohole der allgemeinen Formel I sind auf verschiedenen Wegen erhältlich. So können racemische Aminosäuren oder deren Ester mit diversen hydridischen Reagentien zu den entsprechenden Aminoalkoholen reduziert werden (A. Abiko, S. Masamune, Tetrahedron Lett. 1992, 33, 5517; M. J. McKennon, A. I. Meyers, K. Drauz, M. Schwarm, J. Org. Chem. 1993, 58, 3568 und dort zitierte Literatur).

In manchen Fällen sind diese Verbindungen jedoch nicht oder nicht leicht zugänglich, so z. B. beim (RS)-tert.-Leucin. Dann kann es von Vorteil sein, α-Ketocarbonsäuren oder deren Ester der allgemeinen Formel II, wobei R1 die oben angegebene Bedeutung hat und R2 für Wasserstoff oder einen niederen Alkylrest mit bis zu vier C-Atomen steht, mit Hydroxylamin bzw. einem seiner Salze zum α-Oximinoderivat der allgemeinen Formel III, wobei R1 und R2 die oben angegebenen Bedeutungen haben, umzusetzen und dieses dann 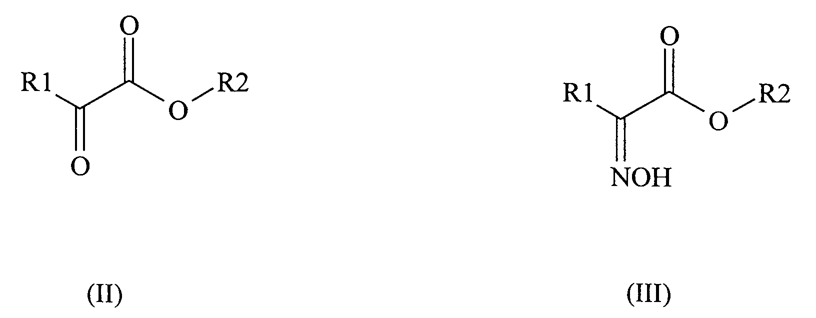 hydridisch zu einem racemischen Aminoalkohol der allgemeinen Formel I zu reduzieren (R. Schröter, in: Houben-Weyl, Methoden der Organischen Chemie, Band XI/1 (Hrsg. E. Müller), S. 495 ff., Georg Thieme Verlag, Stuttgart 1957; R. Hemmer, W. Lürken, in: Houben-Weyl, Methoden der Organischen Chemie, Band E16d (Hrsg. D. Klamann), S. 878 ff., Georg Thieme Verlag, Stuttgart 1992). Als Reduktionsmittel ist hierfür jedoch lediglich Lithiumaluminiumhydrid bekannt, das nicht nur teuer, sondern aufgrund seiner leichten Entflammbarkeit auch sicherheitstechnisch problematisch ist. In einer ähnlichen Reaktionssequenz wurden α-Ketocarbonsäureester in die entsprechenden α-(N-Trimethylsilyl)iminoester überführt, die dann ebenfalls mit Lithiumaluminiumhydroxid zu den racemischen Aminoalkoholen reduziert wurden (Y. Matsuda, S. Tanimoto, T. Okamoto, S. M. Ali, J. Chem. Soc. Perkin Trans. I 1989, 279).

Daher wurde nach einer Möglichkeit gesucht, die Reduktion von einfach herstellbaren α-Oximinocarbonsäuren oder -estern der allgemeinen Formel III mit einem unter Kosten- und Sicherheitsaspekten günstigeren Reduktionsmittel durchzuführen, um so die racemischen Aminoalkohole der allgemeinen Formel I leicht, sauber und in guter Ausbeute zur Verfügung stellen zu können.

Diese Aufgabe wird durch ein Verfahren der eingangs erwähnten Gattung mit den Merkmalen des kennzeichnenden Teils des Anspruchs 1 gelöst. Vorteilhafte Ausgestaltungen der Erfindung werden in den abhängigen Ansprüchen unter Schutz gestellt.

Danach werden die α-Oximinocarbonsäuren oder -ester der allgemeinen Formel III durch Reduktion mit einem Alkaliborhydrid und Schwefelsäure oder auch Chlorwasserstoff als Aktivator in einem Lösungsmittel leicht, sauber und in guter Ausbeute zu den racemischen Aminoalkoholen der allgemeinen Formel I reduziert.

Besonders bevorzugt ist dabei eine Reaktionsvariante, bei der das α-Oximinocarbonsäure-Derivat mit dem Alkaliborhydrid vorgelegt wird und anschließend Chlorwasserstoff oder Schwefelsäure zur Aktivierung der Reduktionsreaktion zugegeben wird. Als Alkaliborhydrid kommt dabei insbesondere das preiswerte Natriumborhydrid, aber auch Lithiumborhydrid in Frage. Pro Äquivalent der α-Oximinoverbindung der allgemeinen Formel III werden dabei 2-5, bevorzugt 3-4 Äquivalente des Alkaliborhydrids zur Reduktion eingesetzt. Für jedes Äquivalent Alkaliborhydrid wird vorteilhaft 1 Äquivalent Chlorwasserstoff oder ½ Äquivalent Schwefelsäure zur Aktivierung eingesetzt. Die bevorzugten Lösungsmittel sind Ether, vor allem solche mit einem Siedepunkt von unter 90°C, insbesondere 1,2-Dimethoxyethan oder Tetrahydrofuran.

Diese Reagentien sind für die Reduktion von Aminosäuren bereits bekannt (Abiko, Masamune 1992). Vorteilhafterweise hat sich herausgestellt, daß sie auch für die Reduktion von α-Oximinocarbonsäuren oder -estern der allgemeinen Formel III zu racemischen Aminoalkoholen der allgemeinen Formel I sehr gut geeignet sind, wobei die Reaktion prinzipiell in einem weiten Temperaturbereich von etwa -20°C bis zur Siedetemperatur des verwendeten Lösungsmittels, bevorzugt aber so durchzuführen ist, daß die Reagentien etwa bei Raumtemperatur, gegebenenfalls unter Kühlung, zusammengegeben werden und die Umsetzung dann durch Erwärmen auf Temperaturen von bis zu etwa 75°C vervollständigt wird. Dies war insbesondere aus dem Grund überraschend, weil das reduzierend wirkende Agens offensichtlich in situ erzeugtes Diboran ist (Abiko, Masamune, 1992), an anderer Stelle (H. Feuer, D. M. Braunstein, J. Org. Chem. 1969, 34, 1817) jedoch darauf hingewiesen wurde, daß Oxime durch Diboran erst bei erhöhter Temperatur von 105-110°C in Diethylenglykoldimethylether-THF zu den korrespondierenden Aminen reduziert werden konnten, während bei 25-65°C keine Reaktion oder bei 85-90°C nur Reduktion zum Hydroxylamin beobachtet wurde. Für die erfindungsgemäße Reduktion von α-Oximinocarbonsäuren oder -estern der allgemeinen Formel III zu racemischen Aminoalkoholen der allgemeinen Formel I kann dagegen überraschenderweise auf derart hohe Temperaturen und den Einsatz des teuren und die Aufarbeitung erschwerenden Lösungsmittels Diethylenglykoldimethylether verzichtet werden. Es ist im Gegenteil sogar vorteilhaft, Ether mit einem Siedepunkt von unter 90°C einzusetzen, da diese erstens preiswerter sind, zweitens die Reduktion unter relativ milden und schonenden Bedingungen durchgeführt werden kann und drittens das Lösungsmittel im Rahmen der Aufarbeitung leicht durch Destillation vom Produkt abgetrennt werden kann.

Nach Beendigung der Reaktion wird das Reaktionsgemisch mit einem Alkohol und/oder Wasser hydrolysiert, das organische Lösungsmittel abdestilliert, der Rückstand in Wasser aufgenommen und mit Salzsäure oder einer anderen Säure sauer gestellt, einige Zeit gerührt, dann, bevorzugt mit Natronlauge, alkalisch gestellt, der racemische Aminoalkohol der allgemeinen Formel I mit einem geeigneten organischen Lösungsmittel extrahiert und nach Einengen erforderlichenfalls durch Destillation, Chromatographie oder Umkristallisation, gegebenenfalls eines Salzes, weiter gereinigt.

Insgesamt eröffnet das erfindungsgemäße Verfahren einen neuen, besonders einfachen Zugang zu racemischen Aminoalkoholen der allgemeinen Formel I durch Reduktion von α-Oximinocarbonsäuren oder -estern der allgemeinen Formel III, die wiederum leicht aus den entsprechenden α-Ketosäuren oder -estern der allgemeinen Formel II hergestellt werden können. Die erfindungsgemäß hergestellten racemischen Aminoalkohole der allgemeinen Formel I dienen u. a. als Startmaterialien für Racematspaltungen, die zu optisch aktiven Aminoalkoholen führen.

Im Rahmen der Erfindung kann (RS)-tert-Leucinol durch Reduktion des leicht zugänglichen Oxims der Trimethylbenztraubensäure erhalten werden. Dieses Oxim kann in bekannter, sehr einfacher Weise (F. Knoop, G. Landmann, Z. Physiol. Chem. 1914, 89, 157) durch Umsetzung von Trimethylbenztraubensäure oder einem ihrer Salze mit Hydroxylamin-hydrochlorid in Wasser hergestellt werden und kristallisiert direkt in guter Ausbeute und hoher Reinheit aus der Reaktionslösung aus.

Die Erfindung wird durch die folgenden Beispiele weiter erläutert:

### Beispiel 1: Herstellung von Trimethylbrenztraubensäureoxim

163 g (1 mol) 93,5 %iges Trimethylbrenztraubensäure-Natriumsalz und 69,5 g (1 mol) Hydroxylaminhydrochlorid wurden bei 40°C in 450 ml Wasser gelöst. Bei langsamem Abkühlen unter Rühren kristallisierte das Produkt aus. Nach 1,5 h Rühren im Eisbad wurden die Kristalle abfiltriert, mit 150 ml Eiswasser gewaschen und zunächst im Vakuum bei 60°C vor- und dann im Vakuumexsikkator über Phosphorpentoxid bis zur Gewichtskonstanz nachgetrocknet. Es wurden 117,8 g (Ausbeute 81 %) Trimethylbrenztraubensäureoxim in Form farbloser Kristalle erhalten.
Schmelzbereich: 120-122°C (Zersetzung) [Lit.: 121°C (F. Knoop, G. Landmann, Z. physiol. Chem. 1914, 89, 157)]

| | | | | |
|---|---|---|---|---|
| C₆H₁₁NO₃ (145,16) | ber. | C 49,64 | H 7,64 | N 9,65 |
| | gef. | C 49,75 | H 7,89 | N 9,71 |

### Beispiel 2: Herstellung von (RS)-tert-Leucinol

Zunächst wurden unter Rühren bei maximal 15°C zu 480 ml 1,2-Dimethoxyethan (DME) 120 ml (2,25 mol) konz. Schwefelsäure getropft.

Zu einer gerührten Suspension von 171 g (4,5 mol) Natriumborhydrid in 1500 ml DME wurden bei 10-30°C portionsweise 218 g (1,5 mol)
Trimethylbrenztraubensäureoxim gegeben, wobei eine heftige Gasentwicklung einsetzte. Anschließend wurde unter Eiskühlung innerhalb von 2,5 h die Schwefelsäure-DME-Lösung zugetropft, wobei die Temperatur von 10°C auf 40°C und nach Entfernen der Kühlung auf 55°C stieg. Danach wurde auf 70°C erhitzt, abgekühlt und der Ansatz 2 Tage bei Raumtemperatur belassen.

Zur Zerstörung überschüssigen Borhydrids wurden zunächst bei 20-55°C 200 ml Methanol und dann 100 ml Wasser zugetropft, wobei die Temperatur bis 60°C stieg. Während des gesamten Hydrolysevorgangs wurde heftige Gasentwicklung beobachtet. Anschließend wurde im Vakuum zum dünnen Brei eingedampft und nach Zugabe von weiteren 500 ml Eiswasser das organische Lösungsmittelgemisch abdestilliert. Nach Zusatz von weiteren 600 ml Wasser wurden bei 25°C 200 ml konz. Salzsäure zugetropft, wobei die Temperatur auf 35°C stieg und erneut heftige Gasentwicklung einsetzte.

Nach 15 min Nachrühren wurde die Suspension mit 1500 ml Toluol versetzt und mit 300 ml 50 %iger Natronlauge alkalisch gestellt. Die dabei auf 55°C gestiegene Temperatur wurde weiter auf 70°C erhöht, worauf die Toluolphase abgetrennt wurde. Die Wasserphase wurde noch zweimal mit je 1 l Toluol bei 70°C extrahiert. Die vereinigten Toluolphasen wurden danach mit Celite behandelt, filtriert und im Vakuum einrotiert, wobei 158 g eines gelblichen Öls erhalten wurden, das in der Kälte kristallisierte. Destillation lieferte 125,1 g (Ausbeute 71 %) (RS)-tert-Leucinol als farblose Flüssigkeit, die bei Raumtemperatur erstarrte. Ein ¹H-NMR-Spektrum bestätigte die vorgeschlagene Struktur.
Siedebereich: 86-95°C / 13 mbar
Schmelzbereich: 34-35°C

| | | | | |
|---|---|---|---|---|
| C₆H₁₅NO (117,19) | ber. | C 61,49 | H 12,90 | N 11,95 |
| | gef. | C 61,10 | H 13,15 | N 11,88 |

### Beispiel 3: Herstellung von 2-Hydroxyimino-4-phenylbuttersäureethylester

103,14 g (0,5 mol) 2-Oxo-4-phenylbuttersäureethylester, 34,75 g (0,5 mol) Hydroxylamin-hydrochlorid, 104 ml (0,75 mol) Triethylamin und 500 ml Ethanol wurden über Nacht bei Raumtemperatur gerührt. Anschließend wurde der Ansatz zur Trockne eingedampft und der Rückstand in 700 ml Methyl-tert-butylether aufgenommen. Nach Waschen mit 200 ml Wasser, 200 ml 0,2 N Salzsäure und 100 ml Wasser wurde die organische Phase zur Trockne eingedampft und der Rückstand nach Filtration aus 170 ml Toluol umkristallisiert. Es wurden 52,9 g (48 %) 2-Hydroxyimino-4-phenylbuttersäureethylester in Form farbloser Kristalle isoliert. Ein ¹H-NMR-Spektrum bestätigte die vorgeschlagene Struktur.
Schmelzbereich: 84-85°C

### Beispiel 4: Herstellung von (RS)-2-Amino-4-phenyl-1-butanol [(RS)-Homophenylalaninol]

In eine Suspension von 24,15 g (0,64 mol) Natriumborhydrid in 200 ml 1,2-Dimethoxyethan (DME) wurden unter Rühren 33,2 g (0,15 mol) 2-Hydroxyimino-4-phenylbuttersäureethylester fest eingetragen. Dazu wurde innerhalb 1 h eine Lösung von 17,2 ml (0,32 mol) konz. Schwefelsäure in 60 ml DME getropft, wobei die Temperatur bei 20-30°C gehalten wurde. Anschließend wurde langsam auf 62°C erwärmt, dann 5 h bei dieser Temperatur gerührt und unter Rühren über Nacht auf Raumtemperatur abgekühlt. Nach vorsichtigem Zusatz von 50 ml Methanol wurde der Ansatz zur Trockne eingedampft, der Rückstand in 120 ml Wasser aufgenommen und mit 25 ml konz. Salzsäure versetzt, wobei heftige Gasentwicklung einsetzte. Nach Zusatz von 150 ml Toluol wurde bis zum Ende der Gasentwicklung gerührt, dann mit 35 ml 50 %iger Natronlauge basisch gestellt, auf 60°C erwärmt, die organische Phase abgetrennt und die wäßrige bei dieser Temperatur nochmals mit 80 ml Toluol extrahiert. Die vereinigten organischen Phasen wurden über Natriumsulfat getrocknet und eingedampft. Als Rückstand verblieben 25,0 g rohes (RS)-2-Amino-4-phenyl-1-butanol in Form eines bräunlichen, zähen Öls. Die Struktur wurde durch ein ¹H-NMR-Spektrum bestätigt.

Weitere Vorteile und Ausführungsformen der Erfindung ergeben sich aus den nachfolgenden Patentansprüchen.

## Patentansprüche

1. Verfahren zur Herstellung racemischer Aminoalkohole der allgemeinen Formel I, 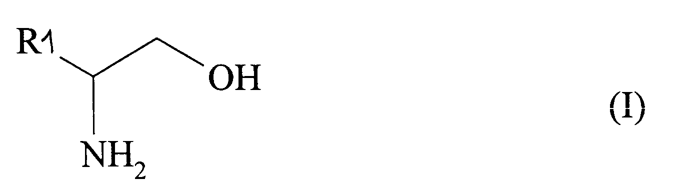 worin R1 ein geradkettiger, verzweigter oder cyclischer Alkyl-, Arylalkyl- oder Arylrest mit bis zu 20 C-Atomen sein kann, der außerdem noch Heteroatome wie O, N oder S enthalten kann,
durch Reduktion von α-Oximinocarbonsäuren oder deren Estern der allgemeinen Formel III, 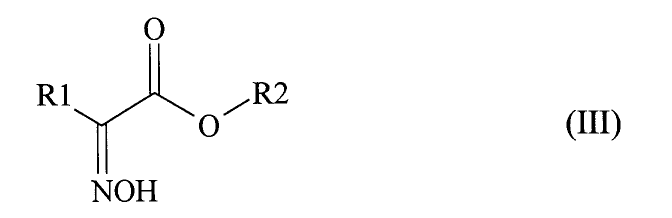 worin R1 die oben angegebene Bedeutung hat und R2 für Wasserstoff oder einen Alkylrest mit bis zu vier C-Atomen steht,
**dadurch gekennzeichnet,**
daß die Reduktion mit einem Alkaliborhydrid in Verbindung mit Chlorwasserstoff oder Schwefelsäure als Aktivator in einem Ether als Lösungsmittel bei -20 °C bis zum Siedepunkt des eingesetzten Lösungsmittels durchgeführt wird.

2. Verfahren nach Anspruch 1,
**dadurch gekennzeichnet,**
daß zur Reduktion die α-Oximinocarbonsäure-Derivat und das Alkaliborhydrid vorgelegt und anschließend Chlorwasserstoff oder Schwefelsäure zur Aktivierung der Reduktionsreaktion zugegeben wird.

3. Verfahren nach Anspruch 1,
**dadurch gekennzeichnet,**
daß als Alkaliborhydrid Natrium- oder Lithiumborhydrid verwendet wird.

4. Verfahren nach einem der vorhergehenden Ansprüche,
**dadurch gekennzeichnet,**
daß das Reaktionsgemisch nach beendeter Reaktion durch Zugabe von Alkohol, Wasser und gegebenenfalls einer Säure hydrolysiert wird, das organische Lösungsmittel abdestilliert wird, die Wasserphase dann alkalisch gestellt wird, der racemische Aminoalkohol der allgemeinen Formel I dann mit einem organischen Lösungsmittel extrahiert und nach Einengen erforderlichenfalls durch Destillation, Chromatographie oder Umkristallisation, gegebenenfalls eines Salzes, weiter gereinigt wird.

## Claims

1. A process for the preparation of racemic aminoalcohols corresponding to the general formula I, 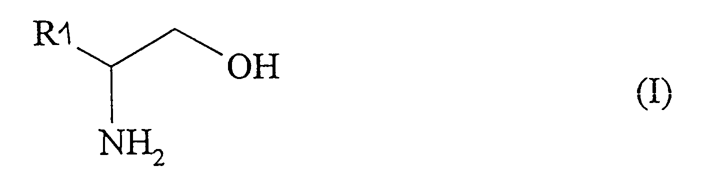 wherein R1 may denote a straight-chain, branched or cyclic alkyl, arylalkyl or aryl group having up to 20 C atoms, which group may contain in addition hetero atoms such as O, N or S,
by reduction of α-oximinocarboxylic acids or the esters thereof corresponding to the general formula III 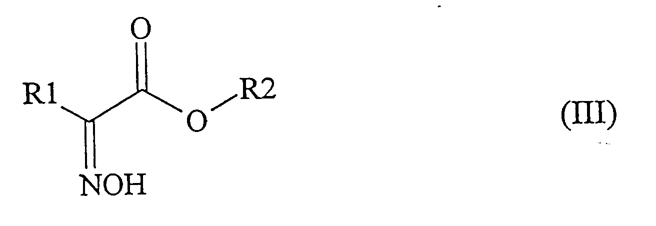 wherein R1 has the meaning given above and R2 represents hydrogen or an alkyl group having up to four C atoms,
characterised in that
the reduction is carried out using an alkali borohydride in combination with hydrogen chloride or sulphuric acid as activator in an ether as solvent at -20°C up to the boiling point of the solvent used.

2. A process according to claim 1,
characterised in that
for the reduction, the α-oximinocarboxylic acid derivative and the alkali borohydride are placed in a reaction vessel and then hydrogen chloride or sulphuric acid is added in order to activate the reducing reaction.

3. A process according to claim 1,
characterised in that
the alkali borohydride used is sodium borohydride or lithium borohydride.

4. A process according to one of the preceding claims,
characterised in that
after the conclusion of the reaction, the reaction mixture is hydrolysed by the addition of alcohol, water and optionally an acid, the organic solvent is distilled off, the aqueous phase is then made alkaline, the racemic aminoalcohol corresponding to the general formula I is then extracted using an organic solvent and, after evaporation to small volume, if necessary is further purified by distillation, by chromatography or by recrystallisation, optionally of a salt.

## Revendications

1. Procédé pour la fabrication d'aminoalcools racémiques de formule générale I, 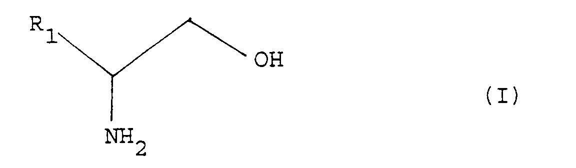 dans laquelle R₁ est un reste alkyle, arylalkyle ou aryle linéaire, ramifié ou cyclique, comportant jusqu'à 20 atomes de carbone, qui peut en outre comporter des hétéroatomes tels que O, N ou S, par réduction d'acides α-oximinocarboxyliques ou de leurs esters de formule générale III 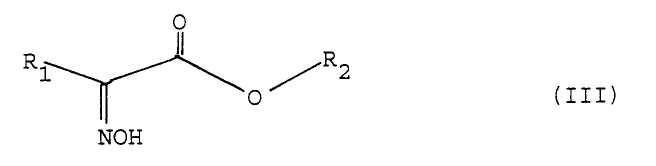 dans laquelle R₁ a la signification indiquée ci-dessus et R₂ représente de l'hydrogène ou un reste alkyle comportant jusqu'à 4 atomes de carbone, caractérisé en ce qu'on réalise la réduction avec un borohydrure alcalin en association avec de l'acide chlorhydrique ou de l'acide sulfurique comme activateur dans un éther comme solvant, entre -20 °C et la température d'ébullition du solvant utilisé.

2. Procédé selon la revendication 1, caractérisé en ce qu'on dispose au préalable, pour la réduction, le dérivé de l'acide α-oximinocarboxylique avec le borohydrure alcalin, puis en ce qu'on ajoute l'acide chlorhydrique ou l'acide sulfurique pour l'activation de la réaction de réduction.

3. Procédé selon la revendication 1, caractérisé en ce qu'on utilise comme borohydrure alcalin du borohydrure de sodium ou de lithium.

4. Procédé selon une quelconque des revendications précédentes, caractérisé en ce qu'à la fin de la réaction, le mélange réactionnel est hydrolysé par addition d'un alcool, de l'eau et, le cas échéant, d'un acide, en ce qu'on élimine le solvant organique par distillation, en ce qu'on règle la phase aqueuse à un pH alcalin, en ce qu'on extrait ensuite l'aminoalcool racémique de formule générale I avec un solvant organique et en ce qu'on le purifie, après concentration, en cas de besoin, par distillation, chromatographie ou recristallisation, le cas échéant d'un sel.
